# EUROPEAN PATENT APPLICATION

(11) **EP 4 652 920 A1**
(43) Date of publication of application: **26.11.2025**
(21) Application number: 24206330.3
(22) Date of filing: 14.10.2024
(51) Int. Cl.: A61B 5/00

(54) **VIRTUAL REALITY-BASED PAIN DIAGNOSIS SYSTEM FOR NEUROPATHIC PAIN**

(30) Priority: 21.05.2024 KR 20240065562
(71) Applicant: Jeonbuk National University Hospital, Jeonju-si Jeonbuk-do 54907 (KR); INDUSTRIAL COOPERATION FOUNDATION JEONBUK NATIONAL UNIVERSITY, Jeonju-si, Jeollabuk-do 54896 (KR)
(72) Inventor: KIM, Gi Wook, 54970 Jeonju-si, Jeonbuk-do (KR); KO, Myoung-Hwan, 54986 Jeonju-si, Jeonbuk-do (KR); KWON, Tae Kyu, 54922 Jeonju-si, Jeonbuk-do (KR); YU, Mi, 55146 Jeonju-si, Jeonbuk-do (KR); KANG, Seung Rok, 54964 Jeonju-si, Jeonbuk-do (KR); KIM, Ra Youn, 55100 Jeonju-si, Jeonbuk-do (KR)
(74) Representative: Cabinet Chaillot

(57) **Abstract**

The present disclosure relates to a virtual reality-based pain diagnosis system for neuropathic pain, and is directed to accurately diagnosing intractable neuropathic pain, which is excruciatingly painful or causes reflex pain responses due to trauma even when there is no direct skin contact or there is very low skin contact. That is, the present disclosure provides a virtual reality-based pain diagnosis system for neuropathic pain, which applies virtual pain stimulation to a patient in a non-contact manner in an extended reality-based fully immersive state and measures a response to the virtual pain stimulation. Therefore, the present disclosure has the effect of accurately diagnosing intractable neuropathic pain by which severe pain is felt or there are reflex pain responses due to trauma, even when there is no direct skin contact or very low skin contact.

## Description

### [Technical Field]

The present disclosure relates to a virtual reality-based pain diagnosis system for neuropathic pain, and more specifically, to a virtual reality-based pain diagnosis system for neuropathic pain that stimulates virtual pain for a non-contact manner to an intractable neuropathic pain patient who experiences hyperesthesia, allodynia, and reflex pain responses in an extended reality (XR)-based fully immersive state and measures a response thereto so as to accurately diagnose intractable neuropathic pain by which the patient complains of physical pain through skin contact, feels extreme pain even with very low skin contact, or experiences reflex pain responses due to trauma.

Meanwhile, the present disclosure relates to a virtual reality-based pain diagnosis system for neuropathic pain, which includes a detected pain imaging unit configured to visualize pain detected in response to virtual stimulation applied through a non-contact immersive virtual stimulation unit so as to be visually confirmed by the diagnosis target, a contact virtual stimulation pain analysis unit configured to analyze the pain detected through the pain detection unit in comparison with stimulation through the contact virtual stimulation unit, a non-contact virtual stimulation pain analysis unit configured to receive and analyze pain induced in response to non-contact immersive virtual stimulation applied to the diagnosis target through the pain detection unit, and a pain diagnosis unit and a pain trend prediction unit configured to perform deep running analysis of pain and trend in response to stimulation of the diagnosis target by comparing and analyzing the pain induced in response to the virtual stimulation applied through the contact virtual stimulation unit and the non-contact immersive virtual stimulation unit, thereby diagnosing and analyzing pain and pain trend in response to skin contact and non-contact stimulation of the diagnosis target through deep learning.

### [Background Art]

In general, neuropathic pain is a nerve pain caused by damage or functional abnormality of the nervous system, and develops into intractable chronic pain through sensitization from the periphery to the central. This is a pain syndrome that significantly reduces the quality of life of patients suffering from this pain due to chronic and long-lasting characteristics and causes not only the pain itself but also social problems such as decreased productivity due to sleep disorders, emotional disorders such as depression, and decreased social adaptability.

The causes of this type of neuropathic pain include nerve damage due to injury or medical problems, or unknown reasons.

The diagnostic methods for neuropathic pain are mostly through direct skin contact, such as the McGill Pain Questionnaire, Visual Analogue Scale (VAS), Activities of Daily Living Scale (ADL Scale), Neuropathic Pain Scale, neurological examination, sensory nerve examination, and functional examination of each cranial nerve.

Most of the existing tests for diagnosing neuropathic pain have been based on mechanical or electrical stimulation through direct skin contact. However, mechanical stimulation may not be possible for neuropathic pain patients. Among neuropathic pain, intractable neuropathic pain is not easily accurately diagnosed because, even when there is no direct skin contact or there is very low skin contact, severe pain is felt or there are reflex pain responses due to trauma.

Therefore, there is a need to develop diagnostic technology using immersive non-contact mixed reality stimulation that allows patients to be fooled into thinking they are receiving real stimulation without direct skin contact.

### [RELATED LITERATURES]

### [Patent Literature]

Korean Unexamined Patent Publication No. 10-2019-0050833

### [Disclosure]

### [Technical Problem]

Accordingly, the present disclosure is directed to solving the problem that intractable neuropathic pain, which is excruciatingly painful or causes reflex pain responses due to trauma even when there is no direct skin contact or there is very low skin contact, is not accurately diagnosed using non-contact mixed reality stimulation.

### [Technical Solution]

That is, the present disclosure provides a virtual reality-based pain diagnosis system for neuropathic pain, which applies virtual pain stimulation to a patient in a non-contact manner in an extended reality-based fully immersive state and measures a response to the virtual pain stimulation.

In the present disclosure, the virtual reality-based pain diagnosis system for neuropathic pain may include a virtual stimulation image data unit configured to store a virtual stimulation image for displaying a virtual stimulation image for the non-contact immersive virtual stimulation on an extended reality display of the non-contact immersive virtual stimulation unit, a pain detection unit configured to detect pain induced to a diagnosis target in response to the virtual stimulation applied through the non-contact immersive virtual stimulation unit, a detected pain imaging unit configured to visualize the pain detected through the pain detection unit so as to be visually confirmed by the diagnosis target, an extended reality display unit configured to display the detected pain imaged through the detected pain imaging unit through extended reality, a non-contact virtual immersive stimulation pain analysis unit configured to analyze the pain detected through the pain detection unit in comparison to stimulation through the non-contact immersive virtual stimulation unit, a non-contact immersive virtual stimulation pain data unit configured to store pain data detected through the pain detection unit and analysis data analyzed through the non-contact immersive virtual stimulation pain analysis unit, a non-contact virtual stimulation pain analysis unit configured to receive the pain, which is induced by non-contact immersive virtual stimulation applied to the diagnosis target through the non-contact immersive virtual stimulation unit, through the pain detection unit and analyze the pain, a pain trend prediction unit configured to perform deep running analysis of a pain trend for the stimulation of the diagnosis target by comparing and analyzing the pain induced by the virtual stimulation applied through the contact virtual stimulation unit and the non-contact immersive virtual stimulation unit, and a pain diagnosis unit configured to diagnose pain through deep running analysis of the pain for the stimulation of the diagnosis target by comparing and analyzing the pain caused by the virtual stimulation applied through the contact virtual stimulation unit and the non-contact immersive virtual stimulation unit.

In the present disclosure, a virtual stimulation image of the non-contact immersive virtual stimulation provided through the non-contact immersive virtual stimulation unit may be provided to the diagnosis target through a virtual stimulation image-related image extended reality display unit related to the virtual stimulation image so that the diagnosis target selects and stimulates a virtual stimulation image to which response sensitivity is sensitive, and the virtual reality-based pain diagnosis system for neuropathic pain may further comprise an image response calculation pain image selection unit configured to detect a heart rate change for the virtual stimulation image-related image provided to the diagnosis target, extract a virtual stimulation image related to the virtual stimulation image that maximizes a heart rate using the virtual stimulation image data unit, and provide the virtual stimulation image to the non-contact immersive virtual stimulation unit.

### [Advantageous Effects]

Therefore, the present disclosure has the effect of accurately diagnosing intractable neuropathic pain by which severe pain is felt or there are reflex pain responses due to trauma, even when there is no direct skin contact or very low skin contact, by including a non-contact immersive virtual stimulation unit capable of applying virtual pain stimulation to a patient in a non-contact manner in an extended reality-based fully immersive state, a non-contact virtual stimulation pain analysis unit configured to receive and analyze pain induced in response to non-contact immersive virtual stimulation applied to the diagnosis target through the pain detection unit, and a pain diagnosis unit and a pain trend prediction unit configured to perform deep running analysis of pain and trend in response to stimulation of the diagnosis target by comparing and analyzing the pain induced in response to the virtual stimulation applied through the contact virtual stimulation unit and the non-contact immersive virtual stimulation unit, thereby diagnosing and analyzing pain and pain trend in response to skin contact and non-contact stimulation of the diagnosis target through deep learning.

### [Description of Drawings]

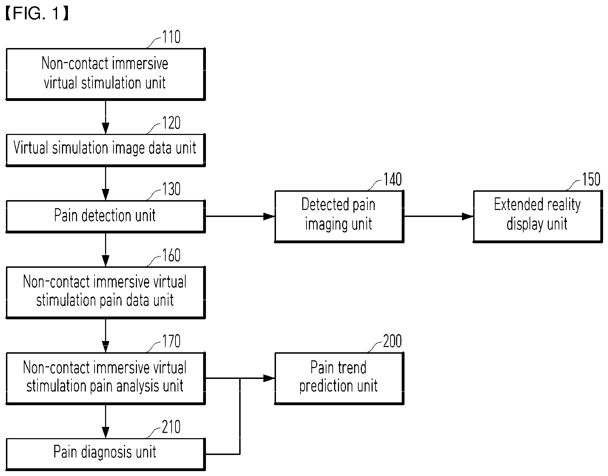

FIG. 1 is a block diagram showing an embodiment of the present disclosure.

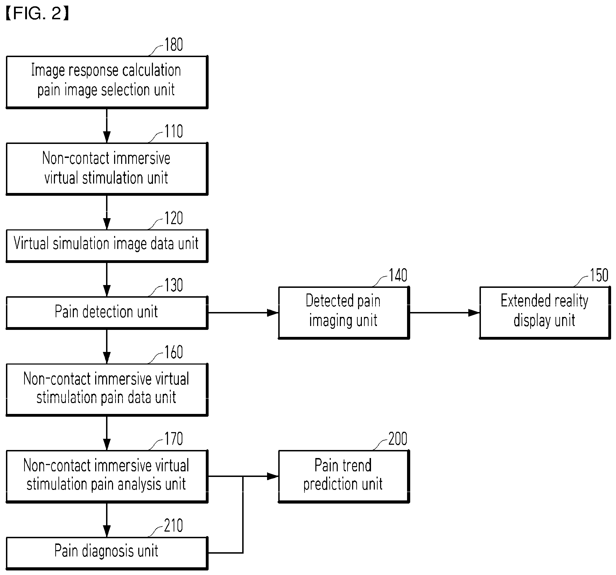

FIG. 2 is a block diagram showing another embodiment of the present disclosure.

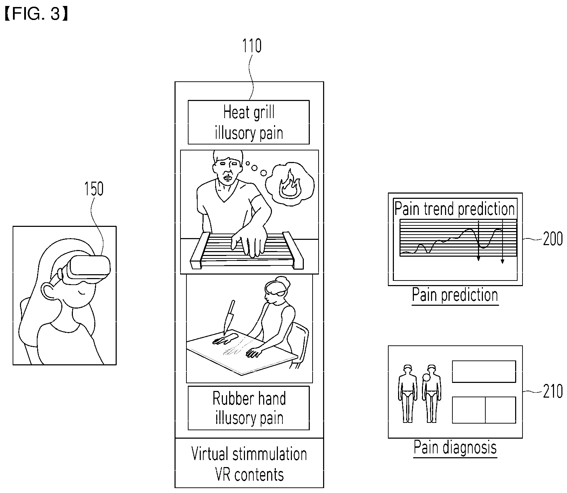

FIG. 3 is a virtual example showing an embodiment of the present disclosure.

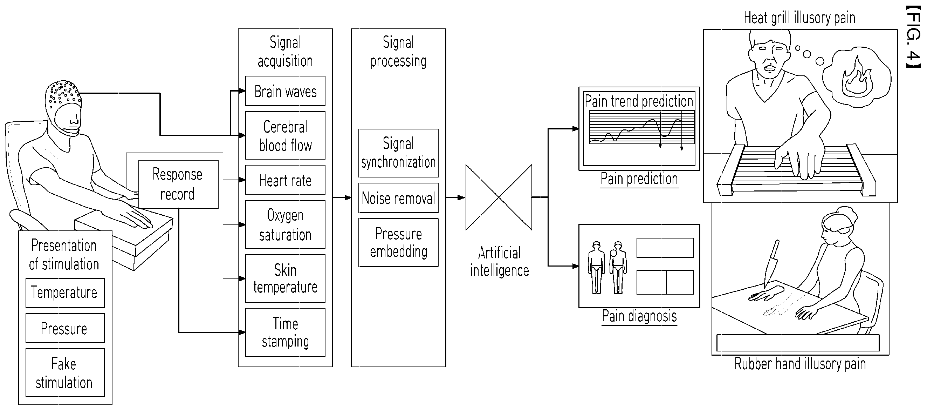

FIG. 4 is a demonstration example and signal processing flowchart of the present disclosure.

### [Best Mode]

Hereinafter, the present disclosure will be described in detail as follows with reference to the attached drawings.

The present disclosure is directed to accurately diagnosing intractable neuropathic pain by which severe pain is felt or there are reflex pain responses due to trauma, even when there is no direct skin contact or very low skin contact. The terms used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation.

Therefore, the description proposed herein is just a preferable example for the purpose of illustrations only, not intended to limit the scope of the disclosure, so it should be understood that other equivalents and modifications could be made thereto without departing from the scope of the disclosure.

That is, the present disclosure provides a virtual reality-based pain diagnosis system for neuropathic pain, which applies virtual pain stimulation in a non-contact manner in an extended reality-based fully immersive state and measures a response to the virtual pain stimulation.

The virtual reality-based pain diagnosis system includes a non-contact immersive virtual stimulation unit 110, a virtual stimulation image data unit 120, a pain detection unit 130, a detected pain imaging unit 140, an extended reality display unit 150, a non-contact immersive virtual stimulation pain data unit 160, a non-contact virtual stimulation pain analysis unit 170, an image response calculation pain image selection unit 180, a pain trend prediction unit 200, and a pain diagnosis unit 210.

Here, the non-contact immersive virtual stimulation unit 110 is provided to induce non-contact immersive virtual stimulation to a diagnosis target through the extended reality display unit.

The non-contact immersive virtual stimulation unit 110 stimulates the diagnosis target with illusory pain, such as rubber hand illusory pain or heat grill illusory pain.

In addition, the virtual stimulation image data unit 120 is provided to store a virtual stimulation image for non-contact immersive virtual stimulation so that the virtual stimulation image may be displayed on an extended reality display of the non-contact immersive virtual stimulation unit.

The virtual stimulation image data unit 120 provides a virtual stimulation image, such as a rubber hand illusion pain image or a heat grill illusion pain image, stored in then-contact immersive virtual stimulation unit.

Also, the pain detection unit 130 is provided to detect pain induced to the diagnosis target in response to the virtual stimulation applied through a contact virtual stimulation unit.

The pain detection unit 130 detects brain waves, cerebral blood flow, heart rate, oxygen saturation, and skin temperature by acquiring them at regular intervals.

The pain signal detected through the pain detection unit 130 is adjusted through signal synchronization, noise removal, and pressure embedding, and then output.

In addition, the detected pain imaging unit 140 is provided to visualize the pain detected through the pain detection unit so as to be visually confirmed by the diagnosis target.

Also, the extended reality display unit 150 is provided to display the detected pain, which is imaged through the detected pain imaging unit, through extended reality.

In addition, the non-contact virtual stimulation pain analysis unit 170 is provided to receive and analyze the pain, which is induced by non-contact immersive virtual stimulation applied to the diagnosis target through the non-contact immersive virtual stimulation unit, through the pain detection unit.

Also, the pain trend prediction unit 200 is provided to perform deep running analysis of a pain trend for stimulation of the diagnosis target by comparing and analyzing the pain induced by the virtual stimulation applied through the contact virtual stimulation unit and the non-contact immersive virtual stimulation unit.

In addition, the pain diagnosis unit 210 is provided to diagnose pain through deep running analysis of the pain for the stimulation of the diagnosis target by comparing and analyzing the pain caused by the virtual stimulation applied through the contact virtual stimulation unit and the non-contact immersive virtual stimulation unit.

Meanwhile, in an embodiment of the present disclosure, the virtual stimulation image of the non-contact immersive virtual stimulation provided through the non-contact immersive virtual stimulation unit 110 is provided to the diagnosis target through the virtual stimulation image-related image extended reality display unit 150 related to the virtual stimulation image so that the diagnosis target may select and stimulate a virtual stimulation image to which response sensitivity of the diagnosis target is sensitive, and the virtual reality-based pain diagnosis system for neuropathic pain may further include an image response calculation pain image selection unit 180 configured to detect a heart rate change for the virtual stimulation image-related image provided to the diagnosis target, extract a virtual stimulation image related to the virtual stimulation image that maximizes a heart rate using the virtual stimulation image data unit 120, and provide the virtual stimulation image to the non-contact immersive virtual stimulation unit 110.

Hereinafter, the effects of the present disclosure will be described.

As described above, the virtual reality-based pain diagnosis system for neuropathic pain, which stimulates virtual pain for a non-contact manner in an extended reality-based fully immersive state and measures a response to the virtual pain stimulation, includes a non-contact immersive virtual stimulation unit 110 configured to induce virtual stimulation to a diagnosis target by skin contact, a pain detection unit 130 configured to detect pain induced to the diagnosis target in response to the virtual stimulation applied through the non-contact immersive virtual stimulation unit, a detected pain imaging unit 140 configured to visualize the pain detected through the pain detection unit so as to be visually confirmed by the diagnosis target, an extended reality display unit 150 configured to display the detected pain imaged through the detected pain imaging unit through extended reality, a non-contact virtual stimulation pain analysis unit 170 configured to receive the pain, which is induced by non-contact immersive virtual stimulation applied to the diagnosis target through the non-contact immersive virtual stimulation unit, through the pain detection unit and analyze the pain, a virtual stimulation image data unit 120 configured to store a virtual stimulation image for displaying a virtual stimulation image for the non-contact immersive virtual stimulation on an extended reality display of the non-contact immersive virtual stimulation unit, a pain trend prediction unit 200 configured to perform deep running analysis of a pain trend for the stimulation of the diagnosis target by comparing and analyzing the pain induced by the virtual stimulation applied through the non-contact immersive virtual stimulation unit, and a pain diagnosis unit 210 configured to diagnose pain through deep running analysis of the pain for the stimulation of the diagnosis target by comparing and analyzing the pain caused by the virtual stimulation applied through the non-contact immersive virtual stimulation unit, thereby diagnosing and analyzing pain and pain trend in response to skin contact and non-contact stimulation of the diagnosis target through deep learning.

### [Reference Symbols]

- 110:: non-contact immersive virtual stimulation unit
- 120:: virtual stimulation image data unit
- 130:: pain detection unit
- 140:: detected pain imaging unit
- 150:: extended reality display unit
- 160:: non-contact immersive virtual stimulation pain data unit
- 170:: non-contact immersive virtual stimulation pain analysis unit
- 180:: image response calculation pain image selection unit
- 200:: pain trend prediction unit
- 210:: pain diagnosis unit

## Claims

1. A virtual reality-based pain diagnosis system for neuropathic pain, which applies virtual pain stimulation to a patient in a non-contact manner in an extended reality-based fully immersive state and measures a response to the virtual pain stimulation.

2. The virtual reality-based pain diagnosis system for neuropathic pain according to claim 1, comprising:
a non-contact immersive virtual stimulation unit configured to induce virtual stimulation to a patient in a non-contact manner in an extended reality state;
a pain detection unit configured to detect pain induced to a diagnosis target in response to the virtual stimulation applied through the non-contact immersive virtual stimulation unit;
a detected pain imaging unit configured to visualize the pain detected through the pain detection unit so as to be visually confirmed by the diagnosis target;
an extended reality display unit configured to display the detected pain imaged through the detected pain imaging unit through extended reality;
a non-contact virtual stimulation pain analysis unit configured to receive the pain induced by non-contact immersive virtual stimulation applied to the diagnosis target through the non-contact immersive virtual stimulation unit, which is provided to induce non-contact immersive virtual stimulation to the diagnosis target through the extended reality display unit, through the pain detection unit and analyze the pain;
a pain trend prediction unit configured to perform deep running analysis of a pain trend for the stimulation of the diagnosis target by comparing and analyzing the pain induced by the virtual stimulation applied through the non-contact immersive virtual stimulation unit; and
a pain diagnosis unit configured to diagnose pain through deep running analysis of the pain for the stimulation of the diagnosis target by comparing and analyzing the pain caused by the virtual stimulation applied through the non-contact immersive virtual stimulation unit.

3. The virtual reality-based pain diagnosis system for neuropathic pain according to claim 2, further comprising:
a virtual stimulation image data unit configured to store a virtual stimulation image for displaying a virtual stimulation image for the non-contact immersive virtual stimulation on an extended reality display of the non-contact immersive virtual stimulation unit.

4. The virtual reality-based pain diagnosis system for neuropathic pain according to claim 2,
wherein a virtual stimulation image of the non-contact immersive virtual stimulation provided through the non-contact immersive virtual stimulation unit is provided to the diagnosis target through a virtual stimulation image-related image extended reality display unit related to the virtual stimulation image so that the diagnosis target selects and stimulates a virtual stimulation image to which response sensitivity is sensitive, and
wherein the virtual reality-based pain diagnosis system for neuropathic pain further comprises an image response calculation pain image selection unit configured to detect a heart rate change for the virtual stimulation image-related image provided to the diagnosis target, extract a virtual stimulation image related to the virtual stimulation image that maximizes a heart rate using the virtual stimulation image data unit, and provide the virtual stimulation image to the non-contact immersive virtual stimulation unit.
